# EUROPEAN PATENT APPLICATION

(11) **EP 4 487 841 A1**
(43) Date of publication of application: **08.01.2025**
(21) Application number: 24169557.6
(22) Date of filing: 10.04.2024
(51) Int. Cl.: A61K 9/70, A61K 9/16, A61L 26/00, A61K 45/06, A61L 15/32, A61L 15/42, A61L 15/44

(54) **ADVANCED DRESSING IN THE FORM OF A COLLAGEN SHEET CONTAINING CHITOSAN AND AZELAIC ACID MICROSPERES FUNCTIONALIZED WITH OZONIZED OIL LOADED MESOPOROUS SILICA**

(30) Priority: 04.07.2023 IT 202300013872
(71) Applicant: Akeso S.r.l., 55100 Lucca (IT)
(72) Inventor: D'ALESSANDRO, Vincenzo, I-55100 LUCCA (IT); BORGHINI, Alice, I-55100 LUCCA (IT); PIETRA, Daniele, I-55100 LUCCA (IT); SPOGLI, Roberto, I-55100 LUCCA (IT); FAFFA, Caterina, I-55100 LUCCA (IT); PASTORI, Gabriele, I-55100 LUCCA (IT)
(74) Representative: Long, Giorgio

(57) **Abstract**

The present invention relates to an advanced dressing in the form of a collagen sheet containing chitosan and azelaic acid microspheres functionalized with ozonized oil loaded on mesoporous silica. In addition, the present invention relates to a process for producing the advanced dressing and use of the advanced dressing in treating skin and mucosal wounds.

## Description

### FIELD OF THE INVENTION

The present invention relates to an advanced dressing, its use in treating skin and mucosal wounds, and a method for producing the same.

### BACKGROUND OF THE INVENTION

A product that promotes the tissue repair process, protecting the wound from infections and maintaining optimal humidity and oxygen permeability conditions of the wound microenvironment is an advanced dressing.

The advanced dressings can be divided into:
Passive - they are for absorbing exudates and protecting the lesion against external agents.
Interactive or active - they play an active role in tissue repair by regulating the lesion microenvironment and ensuring the ideal characteristics in order to facilitate the reparative process.
Bioactive - they interact with the reparative processes, by releasing or forming substances that directly act on healing processes.

In the presence of certain skin and mucosal lesions it can be useful to apply advanced dressings that promote their healing. Lesions that can benefit from application of advanced dressings are skin wounds, ulcers, and sores, such as diabetic ulcers, pressure ulcers and sores (bedsores), general skin ulcers and sores, surgical wounds, cut wounds, puncture wounds, gunshot wounds, abrasions, excoriations, skin rhagades and fissures, chaps, burns.

In particular, **pressure ulcers** are areas of tissue damage of the skin and/or underlying tissues mainly caused by pressure, stretch, or friction. Although widely preventable, this type of damage (also defined as a pressure ulcer, sore, bedsore) represents an important phenomenon in hospital wards and on the territory, both in terms of number of patients involved, and because of time and resources required to treat this problem.

**Lower limb vascular lesions** are defined as those skin wounds with a venous, arterial, and/or mixed vascular aetiology, that are located below the knee down to the foot and lasting at least eight/ten weeks. Chronic leg ulcers represent a very common pathology in the Western world, mainly affecting the elderly, thus determining a high social burden.

**Diabetic ulcers,** in particular those responsible for the diabetic foot, occur when diabetic neuropathy or lower limb arteriopathy compromises the foot function or structure. These two cases, also defined as neuropathic foot or ischemic foot, are profoundly different from each other: however, in the majority of subjects, especially the elderly, they both coexist, leading to the so-called neuroischemic foot.

A **surgical wound** is a solution of continuity of tissues caused by a mechanical agent. In clinical practice, two major types of surgical wounds can be encountered:
- wounds healing by primary intention, wherein flaps have been brought together by applying a suture. They quickly get repaired, typically developing a linear scar, often barely visible;
- wounds healing by secondary intention, wherein flaps are not brought together often because of an infection. Healing is slow, and the scar formed may vary in size.

**Cut wounds** are one of the most common household accidents, and are caused by sharp objects, such as knifes or pieces of glass. They typically appear as linear wounds with clear edges.

**Puncture wounds** are caused by pointed objects (daggers, nails, etc.) which cause lesions developed more in depth than on the surface. In addition to the entrance wound, these wounds have a track extending in depth, and can even become penetrating or perforating wounds when the presence of an exit wound is also observed.

**Gunshot wounds** are caused by projectiles (bullets or fragments) with various shapes and sizes come out from firearms or produced by the explosion of explosive devices. When the projectile is driven by a very high penetrating force, it can exit from the body that has been shot through an exit wound: usually, this appears larger and more jagged than the entrance wound, as the projectile drags tissue fragments with it through its path which increase its sizes.

**Abrasions** are wounds caused by a superficial skin damage, not deeper with respect to epidermis. They are less severe than a laceration, and bleeding, if any, is minimal. The most common abrasions occur when the skin is rubbed against a rough surface. They can be divided into:
- first-degree abrasion: it involves just an epidermal damage;
- second-degree abrasion: it involves epidermis and dermis, and it may bleed slightly;
- third-degree abrasion: it involves damages to the subcutaneous skin layer, and it is often referred to as an avulsion when skin layers are fully removed.

**Excoriations** are superficial skin lesions of traumatic origin that affect epidermis and superficial dermis. Excoriations appear with varying intensity of pain, oedema, skin redness and irritation, and optional bleeding. Depending on the epidermal removal degree, they can be divided into two types of excoriations:
- 1^{st} degree excoriation: the lesion involves the outermost epidermal layer. It is a mild type wound, usually not accompanied by bleeding, with a tendency to form a scar after a few days;
- 2^{nd} degree excoriation: the wound has a deeper nature, it involves dermis as well, and it is often accompanied by bleeding. The scarring occurs within 5-7 days.

**Rhagades** or **fissures** are thin and elongated cuts which are formed at the stratum corneum or more in depth, and are caused by dry skin, insufficient skin nourishment, aggressive detergent use, drug use, and others.

**Chaps** are small cracks that can be formed in various parts of the body. The reasons for their formation are to be looked for mostly in external agents, but in certain cases endogenous factors can also contribute to the appearance of this disturb. In the first instance, chap appearance is promoted by the loss of normal flexibility, and mainly cutaneous hydration. Typically, the loss of cutaneous hydration occurs due to atmospheric agents, such as excessive sun, wind, and cold exposure. However, the chap onset can be linked also to other factors of, for example, traumatic (excessive rubbing), cosmetic (use of aggressive and inadequate products), bacterial (infections), nutritional (vitamin deficiencies, dehydration) origin.

A **burn** is an acute wound of traumatic origin caused by the contact with a flame, hot liquids or solids, chemical substances, electricity, or radiant energy. Based on their depth, they are divided into first- and second-degree superficial, second-degree deep and third-degree; the optimal treatment for the first two is a dressing, which generally allows healing within two weeks; in the case of deep burns, a surgical treatment is the optimal one.

Several substances or products exhibit useful properties for the regeneration of tissues affected by wounds.

Although various attempts (from products commercially available or from scientific and patent literature) to associate various substances trying to obtain dressings effectively promoting a tissue repair process, while protecting from infections and maintaining optimal humidity and oxygen permeability conditions of the wound microenvironment and allowing for exudate absorption are known, there is still a need to be capable of combining different substances with different properties, in such ratios to exhibit synergistic effects in terms of promoting a tissue repair process, protecting from infections, and maintaining optimal humidity and oxygen permeability conditions of the wound microenvironment, while possibly being well-tolerated and easily applied.

In fact, there are various technical issues that should be solved by an ideal advanced dressing. In particular, such dressings should:
- act as a vehicle for active substances,
- exhibit a very low toxicity/cytotoxicity against the damaged tissue during healing, but be active against microorganisms, so as to ensure a microbial load control,
- be well-tolerated, comfortable, and painless,
- be suitable even for sustained and repeated uses,
- be easily applied,
- not require frequent changes,
- not be adherent to the lesion tissue,
- be readily removable/detachable, or be resorbable,
- preserve wound edges,
- control the unpleasant odours that may come from the lesion,
- allow for gas exchange of oxygen, carbon dioxide, and water vapor with the environment,
- be impermeable to the entry of external microorganisms,
- prevent or treat the infection,
- ensure mechanical protection (protect the lesion against possible traumas),
- if possible, be able to absorb the exudate possibly present,
- if possible, allow for monitoring the reparative process without the need to remove the dressing,
- be compatible with other possible medications or treatments to be applied on the wound bed (for example, antimicrobial drugs, analgesics, anti-inflammatory drugs, local anaesthetics, and the like), with therapies such as those having a negative pressure, and with possible secondary dressings used as a supplementation or in order to fix the primary dressings.

Overall, they should therefore have a good compliance by the subjects being treated therewith, for example by providing an efficient dose of various active substances acting synergistically, and physicochemical, mechanical, and biocompatibility characteristics such that frequent applications are not required, i.e., preferably being applied once a week, or less frequently.

### SUMMARY OF THE INVENTION

The above-mentioned aim has been achieved by an advanced dressing in the form of a collagen sheet containing chitosan and azelaic acid microspheres functionalized with ozonized oil loaded on mesoporous silica.

The advanced dressing of the invention is a primary, non-adherent dressing, to be placed in the wound bed, antiseptic, which can be associated with a secondary dressing such as a non-woven gauze, a cotton gauze, and/or a fixing system such as a plaster, or a self-adherent bandage, and the like.

The inventors of the present invention have surprisingly found that said ingredients (collagen, chitosan, azelaic acid, ozonized oil, and mesoporous silica) when formulated in microspheres and incorporated in a collagen porous sheet, show synergistic activity with each other in terms of low adhesiveness (making the dressing painless at the time of change/removal), antimicrobic power (protecting against exogenous infections), stimulation of the tissue regeneration process, exudate absorption, maintenance of optimal humidity and oxygen permeability conditions of the wound microenvironment.

It is therefore an object of the present invention an advanced dressing in the form of a collagen sheet containing chitosan and azelaic acid microspheres functionalized with ozonized oil loaded on mesoporous silica as outlined in the appended claims 1 to 7.

It is another object of the invention a process for producing the advanced dressing, as outlined in the appended claim 8.

It is another object of the invention an advanced dressing in the form of a collagen sheet containing chitosan and azelaic acid microspheres functionalized with ozonized oil loaded on mesoporous silica for use in treating skin and mucosal wounds, as outlined in the appended claims 9 and 10.

In fact, lesions that can particularly benefit from the application of the advanced dressing of the present invention are skin and mucosal wounds such as, but not limited to, diabetic ulcers, pressure ulcers and sores (bedsores), general skin ulcers and sores, surgical wounds, cut wounds, puncture wounds, gunshot wounds, abrasions, excoriations, skin rhagades and fissures, chaps, and burns.

The text of the appended claims forms an integral part of the present description for the purposes of assessing the sufficient disclosure.

Additional characteristics and advantages of the advanced dressings and the process according to the invention will result from the following description of exemplary embodiments, provided by way of indication and not limitation.

### BRIEF DESCRIPTION OF THE DRAWINGS

Figure 1 shows a step of the collagen sheet production according to the preparation procedure from Example 1, wherein the collagen substrate is placed in molds before being freeze-dried.
Figure 2 shows magnified microscope views of the collagen sheet obtained by the preparation procedure from Example 1, at 500X (figure 2A), 1000X (figure 2B), and 5,000X (figure 2C) magnifications.

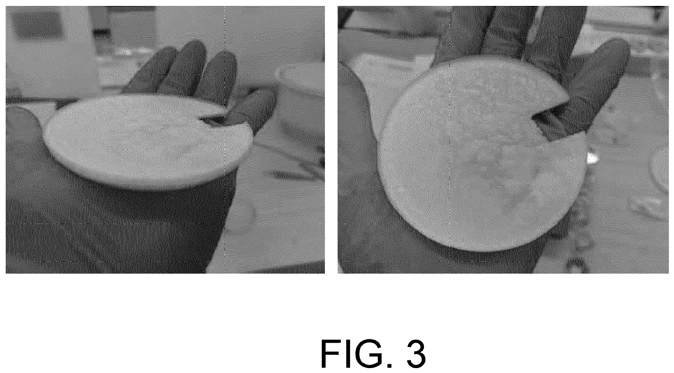

Figure 3 shows a disc-shaped collagen sheet obtained by the preparation procedure from Example 1.

### DETAILED DESCRIPTION OF THE INVENTION

According to a first embodiment, the present invention relates to an advanced dressing in the form of a collagen sheet containing chitosan and azelaic acid microspheres functionalized with ozonized oil loaded on mesoporous silica.

According to certain embodiments of the invention, said advanced dressing is characterized by the following weight percentages, as calculated on the total weight of the dressing:
30 to 70% w/w collagen
15 to 30% w/w chitosan
1 to 10% w/w azelaic acid
10 to 25% w/w silica
2 to 15% ozonized oil.

According to a preferred embodiment of the invention, said advanced dressing is characterized by the following weight percentages, calculated on the total weight of the mixture of said ingredients:
45% to 55% w/w collagen
20 to 25% w/w chitosan
3 to 5% w/w azelaic acid
12 to 18% w/w silica
5% to 10% ozonized oil.

According to a preferred embodiment of the invention, said advanced dressing is characterized by the following weight percentages, calculated on the total weight of the mixture of said components:
50% w/w collagen
23% w/w chitosan
4.1% w/w azelaic acid
16.0% w/w silica
6.9% ozonized oil.

According to a further preferred embodiment of the invention, said advanced dressing has a weight ratio between collagen and chitosan and azelaic acid microspheres functionalized with ozonized oil loaded on mesoporous silica ranging from 50 to 75%.

According to a yet further preferred embodiment of the invention, said advanced dressing is characterized by containing cross-linked collagen, having a porous structure with pores and channels ranging from 50 to 150 microns in size.

According to a yet further preferred embodiment of the invention, said advanced dressing is characterized by an amount of ozonized oil per unit area ranging from 0.05 to 0.5 mg per cm², preferably 0.12 to 0.23 mg per cm².

According to a yet further preferred embodiment of the invention, the chitosan and azelaic acid microspheres functionalized with ozonized oil loaded on mesoporous silica are evenly distributed within the collagen sheet.

According to a yet further preferred embodiment of the invention, the chitosan and azelaic acid microspheres functionalized with ozonized oil loaded on mesoporous silica are not evenly distributed within the collagen sheet, such that a gradient of concentration of the microspheres themselves going from one face of the sheet to the opposite one is formed.

As used herein, the term "advanced dressing" means an advanced dressing suitable for treating skin and mucosal wounds. Primary and secondary advanced dressings exist: the primary ones are placed and act directly on the wound bed, while the secondary ones are used as an integration, or for fixing primary dressings. Non limiting examples of primary dressings are alginates, hydrocolloids, hydrofibers, and polyurethane foams. Non limiting examples of secondary dressings are non-woven gauzes, cotton gauzes, and fixing systems such as plasters or self-adherent bandages.

As used herein, the term "active composition" means a composition of substances that provide a therapeutic effect to the advanced dressing, wherein preferably at least two or more substances have a synergistic effect with each other.

The advanced dressing is preferably in the form of a collagen sheet, functionalized with active substances capable of promoting the tissue repair process, maintaining the optimal humidity and oxygen permeability conditions of the wound microenvironment, preventing the entry of external microorganisms by virtue of antimicrobial activity, and absorbing the wound exudate.

Moreover, the advanced dressing has such physical and mechanical properties as to make it well-tolerated, comfortable, and painless, easy to apply and to remove (by being non-adherent to the tissue involved), or resorbable, not harmful for the wound edges.

As used herein, the term "% by weight" or "% w/w" means the percentage by weight with respect to the total weight of the microspheres.

As used herein, the term "chitosan" means a linear polysaccharide consisting of D-glucosamine and N-acetyl-D-glucosamine, linked by β(1-4) bonds. The chitosan is usually obtained by chitin deacetylation, it generally extracted from crustacean (crabs, shrimps, etc.) exoskeleton with an aqueous sodium hydroxide solution. Alternatively, the chitosan can have a fungal origin, i.e., it is obtained by deacetylating the chitin occurring in fungi, where it represents the major component in the cellular wall.

Some characteristics that distinguish various types of chitosans and are responsible for their different properties are viscosity, degree of acetylation, and molecular weight.

Viscosity is measured in 1% aqueous acetic acid solution and expressed in centipoise (cP). Viscosity is measured on a Brookfield NDJ-1 rotating viscometer usable in a viscosity range from 10 to 100,000 cP. At room temperature, 3.0 g of test sample previously dried at a constant weight at 105±2°C is collected, before being placed in 300 mL of water under stirring. Then, 3.0 g of glacial acetic acid is added, and stirring proceeds for 1 hr or until the sample has been completely dissolved. Then, the rotating viscometer is used for determining the viscosity at 20±1°C.

The degree of acetylation is determined by using the NMR spectroscopy and it ranges between 0% and 40%, while the range related to molecular weight ranges between 3800 and 20,000 Daltons.

For the purposes of the invention, the vegetal chitosan has a viscosity lower than 100 cP, and a degree of acetylation lower than 30%.

As used herein, the term "azelaic acid" means a dicarboxylic acid, i.e. a nonanedioic acid (CAS number 123-99-9). It is a natural substance found in grain, rye, and barley.

As used herein, the term "ozonized oil" means an oil or triglyceride subjected to an ozonization process, wherein ozone (Os) is reacted with the oil. Ozone has antimicrobial, anti-inflammatory, and antioxidant properties, which is why the ozonized oil is often used in medical and cosmetic field. The ozonization of the oil can increase its stability and improve its therapeutic properties. The ozonized oil can be used to treat a variety of skin conditions, including wounds, burns, cutaneous infections, acne, eczemas, and dermatitis. It can also be used as an emollient to hydrate and soften the skin. Non limiting examples of ozonized oils and triglycerides are ozonized sunflower seed oil, ozonized olive oil, ozonized extra virgin olive oil, ozonized coconut oil, ozonized jojoba oil, ozonized argan oil, ozonized hemp oil, ozonized medium chain triglycerides, ozonized unsaturated triglycerides, ozonized long chain triglycerides, ozonized short chain triglycerides, and the like.

As used herein, the term "silica" means a chemical compound consisting of silicon (Si) and oxygen (O) and represented by the chemical formula SiOz. It is one of the most common compounds on Earth and it is widely naturally occurring in the form of quartz, sand, crystals, and rocks. The quartz is the most common form of crystalline silica and it is characterized by a three-dimensional lattice structure. On the other hand, amorphous silica is a form of silica that is lacking a well-defined crystalline structure and it is more common in materials such as glass.

A particular form of silica particularly useful for the purposes of the present invention is the mesoporous silica, that has pores ranging between 2 and 50 nanometers in size. These controlled size pores provide unique properties to mesoporous silica, such as a wide surface area and a high adsorption capacity. The mesoporous silica can be obtained through different synthetic methods, among them the sol-gel method and the surfactant self-assembly method. During the synthesis, silica lattices are formed around surfactant or other organic species micelles, which are then removed to generate pores in silica structure. The pore size can be controlled by varying synthetic parameters, such as the type and concentration of the surfactants used. The porous structure of the mesoporous silica can be used to make materials for controlled release of drugs, cosmetics, and other active compounds. The pores can hold and gradually deliver the molecules, allowing a controlled dosing over time.

As used herein, the term "chitosan and azelaic acid microspheres functionalized with ozonized oil loaded on mesoporous silica" or "chitosan and azelaic acid microcapsules functionalized with ozonized oil loaded on mesoporous silica" means microspheres formed by chitosan cross-linking by means of azelaic acid, serving as a cross-linking agent, and functionalized with silica made mesoporous and loaded with ozonized oil.

As used herein, the term "collagen" means the most abundant protein in the body, where it is mainly concentrated at the level of bones, tendons, cartilages, skin, membranes, and blood vessels. In particular, collagen is one of the major components of the connective tissue. In the skin, collagen contributes to the maintenance of skin firmness, tone, and turgor. It occurs in dermis where, together with elastic fibers and glycosaminoglycans, it forms the three-dimensional structure that supports and sustains the skin, giving it strength and elasticity.

Collagen is a fibrous protein composed of a long amino acid chain. It is characterized by the presence of three polypeptide helices coiled around each other, forming a triple helix structure. The collagen amino acid sequence is characterized by the repetition of three major amino acids: glycine, proline, and hydroxyproline, which are essential for the stability of the triple helix structure of the collagen. The glycine is the most abundant amino acid within collagen, and it provides flexibility to its structure. Proline and hydroxyproline are important for the formation of the chemical bonds that maintain the three-dimensional structure of the collagen.

The collagen is involved in tissue repair and regeneration. During the wound healing process, specialized cells produce collagen in order to reconstruct the damaged tissue. The collagen provides a supporting structure for the cells that migrate in the wound area and contributes to the formation of new tissue.

The most common sources of collagen used in medical and cosmetic field comprise: bovine (extracted from bovine skin, bones, or tendons), swine (obtained from pig skin or tendons), marine (extracted from fish or other marine organism tissues), and human recombinant collagen (produced by using genetic engineering techniques, wherein the genes responsible for producing human collagen are inserted in host cells such as bacteria or yeasts in order to produce collagen).

For the purposes of the present invention, the collagen is of bovine origin.

For the purposes of the present invention, the collagen is cross-linked, so as to maintain a porous structure with pores and channels of around 100 microns in size, suitable to accommodate the microspheres and absorb the exudate possibly present in the wound. In fact, the porosity of the sheet promotes the exudate absorption and the subsequent interaction between the exudate and the microcapsules with the activation of chitosan, azelaic acid, and ozonized oil release.

The ingredients of the present dressing are all known and individually available on the market.

The advanced dressing of the invention can also comprise additional ingredients and excipients such as, but not limited to, local anaesthetic active substances, analgesic and anti-inflammatory active substances, antimicrobial active substances, growth factors, cytokines, hormones, vitamins, minerals, vegetal extracts and oils, and the like.

There are no particular restrictions regarding the shape and sizes of the advanced dressing of the invention, which can take, for example, the form of a solid, in particular a disc, cube, parallelepiped, cylinder, sphere, and the like, with the three spatial dimensions x,y,z ranging from 1 mm to 200 mm and a density of 5-15 mg/cm³, preferably 7-10 mg/cm³.

### EXAMPLES

### Example 1 - Preparing a collagen sheet containing chitosan and azelaic acid microspheres functionalized with ozonized oil loaded on mesoporous silica

### Step 1 - Synthesizing chitosan and azelaic acid microspheres

Chitosan and azelaic acid microspheres functionalized with ozonized oil loaded on mesoporous silica were prepared as follows:
in a reactor of cased borosilicate glass, a 5 L aqueous dispersion containing chitosan and azelaic acid was prepared, thermostated at 55°C for 30 mins, then glacial acetic acid was added. The suspension was maintained under vigorous stirring at 55°C for 15 hours, at the end of which a colloidal yellow suspension is obtained.

Before atomization, the phase containing the ozonized oil loaded on silica was prepared according to the following passages:

### Step 2 - Synthesizing mesoporous silica

Silica synthesis provides for using a sol-gel process by using a templating agent (CTAB, cetyl trimethyl ammonium bromide) and a source of silicon (TEOS, tetraethyl orthosilicate) in a very low concentration basic aqueous solution. After heating, the silica precipitates within surfactant micelles forming mesoporous microparticles. Then, these are calcinated in order to remove the surfactant and free the channels.

200 g of CTAB was dissolved in basic aqueous solution, consisting of 95 L of deionized water and 1M NaOH. The resulting mixture was vigorously mixed under heating in the production reactor. Once the temperature of 80°C was reached, 1 L of TEOS was added, and the reaction mixture was maintained at this temperature under stirring for 2 hrs in the reactor. Measured pH = 12.17. At the end of the reaction, the mixture was allowed to cool, then it was collected from the reactor, and left decanting in pails for 6 days, then the supernatant was discarded by centrifugation. The wet product was washed twice in a water/ethanol mixture = 1/1 (4 L per washing), followed by an additional washing in water only. The foam on the surface was removed manually. The product was calcinated in a muffle furnace at 600°C for 5 hours, after which it was used for the next synthesis steps.

### Step 3 - Incorporating ozonized oil in mesoporous silica

In order to incorporate the active ozonized sunflower seed oil into mesoporous silica, a procedure has been selected which provides for the ozonized oil solubilization in a suitable low-boiling solvent and under a high vapor pressure, the mesoporous silica blending with the ozonized oil solution and the subsequent solvent removal at low temperature and reduced pressure while stirring silica (conditions obtained by using a rotary evaporator).

Preliminary tests for solubility evaluation of the ozonized oil have shown that the product is insoluble in methanol and ethanol, but highly soluble in dichloromethane (CH₂Cl₂), so this solvent is selected for the loading procedure.

### Procedure A

3 g of ozonized oil was solubilized in 30 mL of dichloromethane in a flask under magnetic stirring, then 7 g of mesoporous silica calcinated at 600°C was added, and that was all maintained under magnetic stirring for 15 mins. The solvent was evaporated onto a rotary evaporator (15 mTor) at 30°C until a flowing powder was obtained, then at 40°C for further 15 mins.

### Procedure B

15 g of ozonized oil was solubilized in 400 mL of dichloromethane, then 35 g of mesoporous silica calcinated at 600°C (batch 22FL06) was added, and that was all homogenized by Ultra-Turrax for 15 mins, then the solvent was evaporated onto a rotary evaporator as in procedure A.

### Step 4 - Functionalizing the chitosan and azelaic acid microspheres with ozonized oil loaded on mesoporous silica

The phase containing the ozonized oil loaded on silica was poured in the reactor containing the chitosan and azelaic acid microspheres and first dispersed under mechanical stirring for 10 mins at 65°C, and then transferred into a vessel and further dispersed by Ultra-Turrax homogeniser for additional 15 mins.

The so-prepared, light yellow phase was then atomized with an inlet temperature of 220-275°C and an outlet temperature ranging between 104 and 80°C. The atomiser feed rate was of 1.5-2.5 L/hr.

After atomization, the batch was sieved through a 75-micron screen.

### Step 5 - Preparing the collagen sheet

The collagen sheet was prepared according to a modification to the procedure described by Doillon [Doillon CJ, Whyne CF, Brandwein S, Silver FH. Collagen-based wound dressings: control of the pore structure and morphology. J Biomed Mater Res. 1986 Oct;20(8) :1219-28] . Doillon's optimized procedure provides for making a 0.5% w/w hydrolyzed collagen solution and stabilized to pH 3.0 with 0.5M hydrochloric acid, freezing it between -20 and -30°C for 3 hrs and subsequently freeze-drying it in molds for about 17 hours. The so-obtained collagen is not cross-linked and has an average pore size of 100 ± 50 microns. It contains some channels, and such a morphology is ideal for the internal growth of dermal tissue. It was hot cross-linked (thermal cross-linking) in a vacuum oven at 105°C for 24 hours.

### Step 6 - Loading the collagen sheet

After cross-linking, the substrate was taken up with an aqueous suspension containing the microcapsules obtained from the previous steps, frozen at -20°C and freeze-dried one more time. This expedient is suitable for microcapsule insertion within the channels after cross-linking, in fact, in such a way the microcapsules are not subjected to thermal stress.

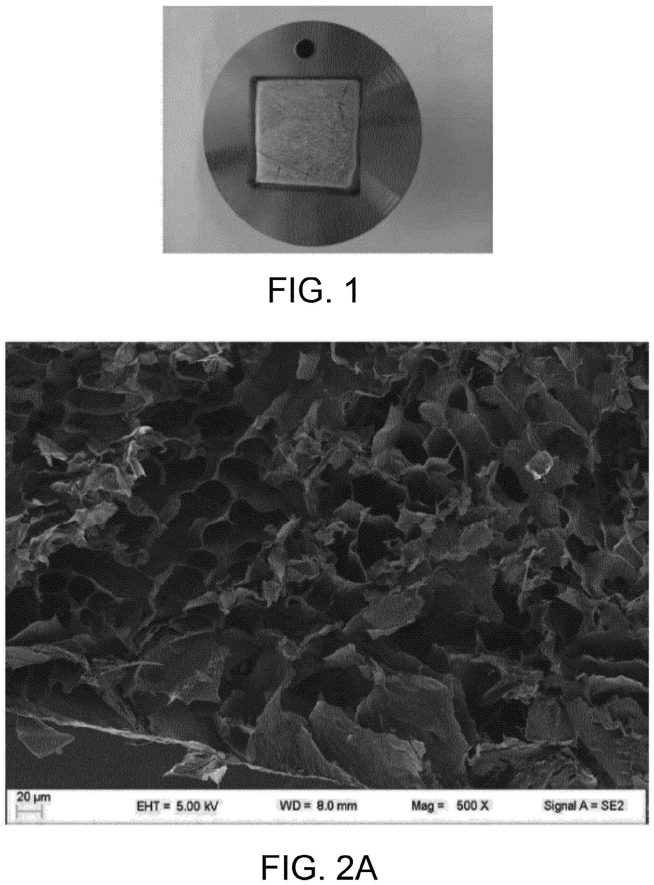

Figure 1 shows a disc-shaped collagen sheet obtained by said preparation procedure.

### Example 2 - Characterizing the collagen sheet containing chitosan and azelaic acid microspheres functionalized with ozonized oil loaded on mesoporous silica

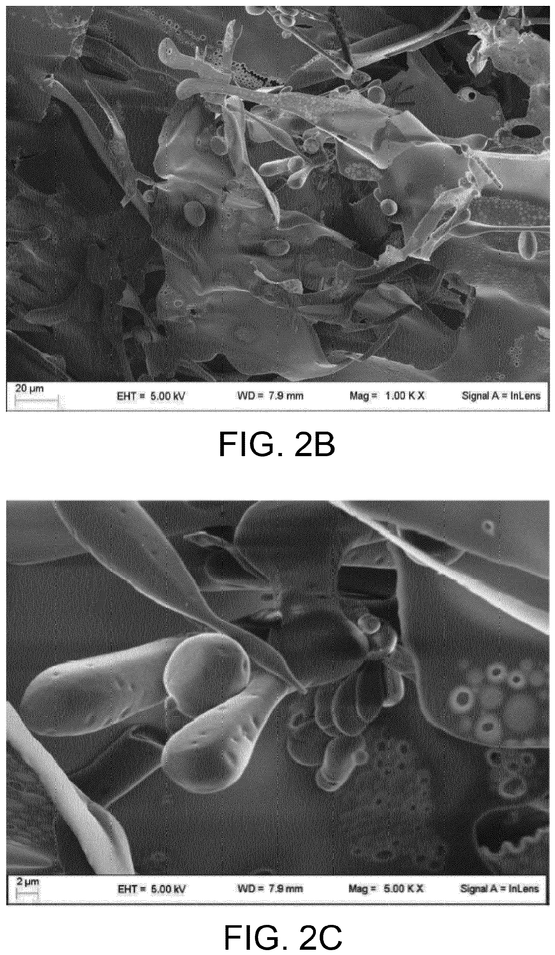

The microspheres obtained according to the procedure from Example 1 appear as a light yellow, almost odourless powder. They have been characterized based on its morphology. This was obtained by a scanning electron microscope (SEM) by using LEO 1525 FE-SEM instrument with EDX probe Bruker Field Emission Scanning Electron Microscope, with magnifications from 500X to 5,000X. The images obtained by SEM (see figures 2A-2C) show a porous structure.

## Claims

1. An advanced dressing in the form of a collagen sheet containing chitosan and azelaic acid microspheres functionalized with ozonized oil loaded on mesoporous silica.

2. The advanced dressing according to claim 1, wherein the active composition is **characterized by** the following weight percentages, based on the total weight of the dressing:
30 to 70% w/w, preferably 45-55%, or about 50% collagen,
15 to 30% w/w, preferably 20-25%, or about 23% chitosan,
1 to 10% w/w, preferably 3-5%, or about 4.1% azelaic acid,
10 to 25% w/w, preferably 12-18%, or about 16.0% silica,
2 to 15%, preferably 5-10%, or about 6.9% ozonized oil.

3. The advanced dressing according to any one of claims 1-2, wherein the weight ratio between collagen and chitosan and azelaic acid microspheres functionalized with ozonized oil loaded on mesoporous silica is from 50 to 75%.

4. The advanced dressing according to any one of claims 1-3, wherein the chitosan is of fungal origin.

5. The advanced dressing according to any one of claims 1-4, wherein the collagen is cross-linked, so as to maintain a porous structure with pores and tunnels ranging from 50 to 150 microns in size.

6. The advanced dressing according to any one of claims 1-5, wherein the amount of ozonized oil per unit area ranges from 0.05 to 0.5 mg per cm², preferably from 0.12 to 0.23 mg per cm².

7. The advanced dressing according to any one of claims 1-6, wherein the dressing has the shape of a solid, in particular a disc, a parallelepiped, a cube, a cylinder, a sphere, and the like, with the three spatial dimensions x,y,z ranging from 1 mm to 200 mm and a density of 5-15 mg/cm³, preferably 7-10 mg/cm³.

8. A process for producing the advanced dressing according to any one of claims 1-7, said process comprising the following steps:
*Step 1* - *Synthesizing chitosan and azelaic acid microspheres*
i) Water dispersing chitosan and azelaic acid;
ii) adding an aqueous glacial acetic acid solution;
iii) vigorously stirring the reaction mixture;
iv) heating the reaction mixture;
v) spray-drying the obtained solution; and
vi) recovering the powder for the subsequent processing steps;
*Step 2* - *Synthesizing mesoporous silica*
i) Preparing the sol-gel solution containing a silica precursor mixed with suitable solvents and optional catalysts or modifiers in order to control the properties of the final material;
ii) condensing by treating the sol-gel solution with a base or acid in order to initiate the condensation of silica precursors and induce the solution formation of silica lattices;
iii) aggregating and forming pores by adding cationic or anionic surfactants to the solution, which form micelles organized in regular structures and serve as a template for pore generation;
iv) ageing the sol-gel solution to allow for pore growth and structure solidification;
v) removing the surfactants by thermal treatments or washings with solvents in order to obtain the final mesoporous silica;
vi) washing and drying the obtained material in order to remove possible residual impurities and humidity;
*Step 3* - *Incorporating ozonized oil within mesoporous silica*
i) Solubilizing ozonized oil in a suitable low-boiling solvent and under high vapor pressure;
ii) blending the mesoporous silica with the ozonized oil solution;
iii) removing the solvent at low temperature and reduced pressure;
iv) recovering the powder for the subsequent processing steps;
*Step 4* - Functionalizing *chitosan and azelaic acid microspheres with ozonized oil loaded on mesoporous silica*
i) Transferring the phase containing the ozonized oil loaded on silica to the reactor containing the chitosan and azelaic acid microspheres and stirring the dispersion;
ii) *homogenizing* the dispersion;
iii) spray-drying;
iv) sieving the obtained powder.
*Step 5* - *Preparing the collagen sheet*
i) Preparing a hydrolyzed collagen solution at the intended percentage and stabilizing it at acidic pH with HCl;
ii) inserting the solution in molds and freezing it at -20°C;
iii) freeze-drying;
iv) thermal cross-linking in a vacuum oven at 105°C for 24 hours;
*Step 6* - *Loading the collagen sheet*
i) Taking up the substrate on the mold with an aqueous microsphere suspension;
ii) freezing at -20°C;
iii) freeze-drying a second time.

9. The advanced dressing according to any one of claims 1-7 for use in treating skin and mucosal wounds.

10. The advanced dressing according to claim 9, wherein the skin and mucosal wounds are selected from the group consisting of: skin wounds, ulcers, and sores, such as diabetic ulcers, pressure ulcers and sores (bedsores), general skin ulcers and sores, surgical wounds, cut wounds, puncture wounds, gunshot wounds, abrasions, excoriations, skin rhagades and fissures, chaps, burns.
